# FASCICULE DE BREVET EUROPEEN

(11) **EP 1 009 406 B1**
(45) Date de publication et mention de la délivrance du brevet: **12.12.2001**
(21) Numéro de dépôt: 98909547.6
(22) Date de dépôt: 17.02.1998
(51) Int. Cl.: A61K 31/55, A61K 9/00

(54) **COMPOSITION PHARMACEUTIQUE POUR L'ADMINISTRATION DE THIOCOLCHICOSIDE A TRAVERS LA MUQUEUSE BUCCALE**
ARZNEIZUBEREITUNG ZUR VERABREICHUNG VON THIOCOLCHICOSID DURCH DIE MUNDSCHLEIMHAUT
PHARMACEUTICAL COMPOSITION FOR THE ADMINISTRATION OF THIOCOLCHICOSIDE VIA THE BUCCAL MUCOSA

(30) Priorité: 20.02.1997 FR 9701994
(43) Date de publication de la demande: 21.06.2000
(73) Titulaire: SANOFI-SYNTHELABO, 75013 Paris (FR)
(72) Inventeur: COLOMBO, Paolo, I-43100 Parma (IT); SANTI, Patrizia, I-43039 Salsomaggiore Terme (IT); ARTUSI, Mariella, I-43100 Parma (IT)
(74) Mandataire: Thouret-Lemaitre, Elisabeth
(86) Numéro de dépôt international: FR9800296
(87) Numéro de publication internationale: WO9836751

(56) Documents cités:
- WO-A-96/41635
- WO-A-97/01570
- FR-A- 2 725 134
- CHEMICAL ABSTRACTS, vol. 124, no. 26, 24 juin 1996 Columbus, Ohio, US; abstract no. 352533, XP002046261 & E. PERUCCA ET AL.: "COMPARATIVE PHARMACOKINETICS AND BIOAVAILABILITYOF TWO ORAL FORMULATIONS OF THIOCOLCHICOSIDE,A GABA-MIMETIC MUSCLE RELAXANT DRUG,IN NORMAL VOLUNTEERS" EUR. J. DRUG METAB. PHARMACOKINET., vol. 20, no. 4, 1995, pages 301-305,
- CHEMICAL ABSTRACTS, vol. 120, no. 17, 25 avril 1994 Columbus, Ohio, US; abstract no. 207873, XP002046262 & P. SANDOUK ET AL.: "SINGLE-DOSE BIOAVAILABILITY OF ORAL AND INTRAMUSCULAR THIOCOLCHICOSIDEIN HEALTHY VOLUNTEERS" BIOPHARM. DRUG DISPOS., vol. 15, no. 1, 1994, pages 87-92,

## Description

La présente invention concerne de nouvelles compositions pharmaceutiques solides pour l'administration de thiocolchicoside à travers la muqueuse buccale.

Le thiocolchicoside peut être utilisé dans le traitement d'appoint des contractures musculaires douloureuses.

Le médicament thiocolchicoside est habituellement administré par voie injectable ou par voie orale. Les données disponibles en littérature montrent une faible biodisponibilité du thiocolchicoside après administration orale, ainsi qu'une variabilité individuelle marquée. Le phénomène peut être attribué, principalement à un effet de premier passage hépatique et au cycle entéro-hépatique.

Les compositions pharmaceutiques solides pour l'administration de thiocolochicoside à travers la muqueuse buccale, objets de la présente invention, permettent de remédier à ces inconvénients. En effet, la muqueuse buccale, en raison de son extension et sa vascularisation, se prête bien à l'absorption du principe actif, à condition que ce dernier soit maintenu au contact étroit avec elle par une composition pharmaceutique appropriée.

La présente invention a pour objet deux compositions pharmaceutiques appropriées pour l'administration du thiocolchicoside à travers la muqueuse buccale. Ces compositions sont de préférence hydrosolubles et sont capables de se dissoudre dans une petite quantité de liquide physiologique (salive) de façon à donner lieu rapidement à une solution dense à concentration élevée de thiocolchicoside au niveau du site d'application, c'est à dire la muqueuse buccale. Elles assurent des niveaux hématiques suffisants pour assurer l'effet thérapeutique recherché et présentent les caractéristiques avantageuses suivantes par rapport à une administration orale de thiocolchicoside :
- une concentration élevée du thiocolchicoside au site d'application ;
- un effet thérapeutique plus rapide ; et
- une biodisponibilité accrue du thiocolchicoside.

La première de ces compositions permet la libération immédiate du thiocolchicoside. Elle se base sur la dissolution rapide de la composition pharmaceutique qui est, de préférence, appliquée dans la région sublinguale. Une partie de la substance active est absorbée localement et une autre est ingérée dans le cycle salivaire.

La deuxième composition pharmaceutique selon l'invention, permet une libération prolongée dans le temps. Cette composition privilégie l'absorption buccale et réduit la quantité ingérée dans le cycle salivaire. De préférence, cette composition pharmaceutique est positionnée dans le sillon entre la gencive et la partie intérieure de la lèvre, de préférence la lèvre supérieure, de façon à éviter une salivation excessive. Dans cette position, pendant un délai prolongé, elle est en mesure de libérer lentement le principe actif et d'assurer la libération prolongée. Cette composition pharmaceutique se caractérise par une hydratation rapide avec formation d'une masse gélifiée dotée d'une haute viscosité et d'une forte adhésivité à la muqueuse suivie de sa lente dissolution.

Les compositions pharmaceutiques selon l'invention sont solides et peuvent comprendre du thiocolchicoside de 4 à 20 mg par unité. De préférence, la quantité de thiocolchicoside varie de 4 à 8 mg par unité. Les compositions selon l'invention comprennent de la gélatine dans des proportions de 10 à 50% en masse, ainsi qu'éventuellement d'autres excipients acceptables au plan pharmaceutique.

Ces derniers sont par exemple des excipients solubles ou des excipients gélifiables.

En plus de ces excipients solubles ou gélifiables, les compositions pharmaceutiques peuvent comprendre des masqueurs de goût tels que par exemple l'aspartame, la saccharine, les cyclamates, les aromes ou l'acide citrique.

La composition pharmaceutique à libération immédiate comprend notamment du thiocolchicoside et de la gélatine, dans les quantités et proportions indiquées précédemment. Les proportions en gélatine varient de préférence de 10 à 35% en masse. Cette composition comprend également un ou plusieurs excipients solubles capables de se dissoudre rapidement dans les liquides physiologiques. Ces excipients solubles présentent, en général, une solubilité supérieure à 20% (g/100 ml).
Ces excipients solubles sont par exemple la glycine ou des sucres tels que le mannitol, le sorbitol, le xylitol ou le glucose. Les proportions en ces excipients solubles varient de 30 à 70% en masse. De préférence, les proportions se situent entre 40 et 70% en masse.

La composition pharmaceutique à libération prolongée comprend notamment du thiocolchicoside dans les quantités indiquées précédemment et de la gélatine dans des proportions de 10 à 50% en masse. Les proportions en gélatine varient de préférence de 25 à 50% en masse. Cette composition comprend également un ou plusieurs excipients gélifiables capables de s'hydrater rapidement et de développer des propriétés bioadhésives. Ces excipients gélifiables présentent, en général, une viscosité supérieure à 100 mPa.s (milliPascal par seconde), mesurée pour une solution aqueuse à 1% (g/100 ml) à 25 °C.
Ces excipients gélifiables sont par exemple des composés macromoléculaires solubles, d'origine naturelle ou synthétique, tels que le collagène, la polyvinylpyrolidone, le polyéthylèneglycole, l'amidon soluble, les dérivés de la cellulose (carboxyméthylcellulose sodique, hydroxypropyléthylcellulose et hydroxypropylméthylcellulose), la gomme de xanthane, la gomme de guar, l'alginate de sodium, les alcools polyvinyliques et la β-cyclodextrine. Les proportions en ces excipients gélifiables varient de 25 à 55% en masse. De préférence, les proportions se situent entre 30 et 50% en masse.

Les compositions pharmaceutiques qui permettent de réaliser l'invention peuvent se présenter sous forme de disques solides qui peuvent être préparés par lyophilisation ou par lyophilisation suivie d'une compression.

Par exemple, les compositions pharmaceutiques de la présente invention peuvent être obtenues soit par lyophilisation directe de la solution contentant les différents composants dans des bacs ayant des cavités préformées de la dimension désirée, soit en comprimant la poudre que l'on obtient après lyophilisation de la solution en vrac, en broyant le lyophilisé dans un mortier et en le tamisant à travers des tamis à la dimension désirée.

De préférence, les compositions pharmaceutiques se présentent sous la forme d'un disque d'un diamètre de 12 à 16 mm et d'une épaisseur de 2 à 4 mm.

Les exemples suivants illustrent l'invention.

### Exemple 1 : Composition pharmaceutique à libération immédiate

### a) Compositions

* Pour une disque à 4 mg de thiocolchicoside obtenu par lyophilisation
   Thiocolchicoside : 4 mg
   Gélatine : 14 mg
   Glycine : 14 mg
   Mannitol : 20 mg
   Aspartame : 0,24 mg
   Acide citrique : 1 mg
   Arome framboise : 0,5 mg
* Pour un disque à 8 mg de thiocolchicoside obtenu par lyophilisation
   Thiocolchicoside : 8 mg
   Gélatine : 10 mg
   Glycine : 14 mg
   Mannitol : 20 mg
   Aspartame : 0,5 mg
   Acide citrique : 1 mg
   Arome framboise : 1 mg

### b) Préparation de 50 disques à 4 mg de thiocolchicoside

La gélatine (700 mg) est dissoute dans 20 ml d'eau sous agitation dans un bêcher à la température d'environ 65°C. Après refroidissement de la solution, sont ajoutés successivement le thiocolchicoside (200 mg), le mannitol (1 g), la glycine (700 mg), l'aspartame (12 mg), l'acide citrique (50 mg) et l'arome framboise (25 mg). La solution ainsi obtenue est transférée dans un ballon étalonné de 25 ml et portée à volume d'eau avec de l'eau distillée. 0,5 ml de cette solution contenant 4 mg de thiocolchicoside, sont versés dans des bacs ayant des creux d'une surface de 1,5 cm². Les bacs sont refroidis à 5°C pendant une heure environ, ils sont gardés ensuite à une température de -20°C pendant 12 heures et sont mis au lyophilisateur.

### Exemple 2 : Composition pharmaceutique à libération prolongée dans le temps

### a) compositions

* Pour un disque à 4 mg de thiocolchicoside obtenu par lyophilisation
   Thiocolchicoside 4 mg
   Gélatine : 14 mg
   Carboxyméthyle cellulose sodique : 12 mg
   Aspartame : 0,3 mg
   Acide citrique : 1 mg
   Arome framboise : 0,5 mg
* Pour un disque à 8 mg de thiocolchicoside obtenu par lyophilisation
   Thiocolchicoside : 8 mg
   Gélatine : 10 mg
   Carboxyméthyle cellulose : 12 mg
   Aspartame : 0,4 mg
   Acide citrique : 1 mg
   Arome framboise : 1 mg

### b) Préparation pour 50 disques de 4 mg de thiocolchicoside

La gélatine est dissoute (700 mg) dans un volume suffisant d'eau sous agitation à la température d'environ 65°C dans un bêcher. A la solution ainsi obtenue est incorporée graduellement, tout en remuant, la carboxyméthylcellulose sodique (600 mg). Après dissolution totale, le thiocolchicoside (200 mg) suivi de l'aspartame (20 mg), de l'acide citrique (50 mg) et de l'arome framboise (25 mg) sont ajoutés. La solution est ensuite portée à volume dans un ballon étalonné de 25 ml avec de l'eau distillée. 0,5 ml de solution, contenant 4 mg de thiocolchicoside est versée dans des bacs creux d'une surface de 1,5 cm². Les bacs sont refroidis pendant une heure environ, gardés à une température de -20°C pendant 12 heures et lyophilisés.

### Exemple 3 : Essais d'imprégnation in vitro des compositions à libération immédiate et à libération prolongée dans le temps

La muqueuse buccale porcine dont la ressemblance avec la muqueuse humaine a été confirmée par des études histologiques, est utilisée.

La muqueuse porcine utilisée comprend une couche de muqueuse mais également une partie du tissu connectif sous-jacent et du tissu musculaire. L'essai évalue la vitesse de disparition du thiocolchicoside de la composition pharmaceutique.

Des cellules verticales de diffusion en verre d'une surface de 3,91 cm² (Franz) sont utilisées. L'échantillon de muqueuse isolé quelques heures après l'abattage du porc est monté horizontalement entre le compartiment donneur, destiné à recevoir la composition pharmaceutique, et le compartiment récepteur destiné à recevoir la muqueuse tournée vers le compartiment donneur. Le compartiment récepteur est rempli de 19 ml de solution physiologique dégazée. Dans le compartiment donneur est introduite une composition lyophilisée contenant 4 mg de thiocolchicoside à laquelle est ajoutée 20 µl de solution physiologique dégazée ; le tout est ensuite recouvert de papier filtre imbibé de solution physiologique et fermé avec de la parapellicule. Les cellules sont immergées dans un bain thermostaté à 37°C et basculant. Après des intervalles déterminés différents pour chaque cellule (15, 30, 45 ou 60 minutes), le résidu de composition présent dans le compartiment donneur est enlevé et la muqueuse est lavée soigneusement avec de l'eau distillée.

Les échantillons obtenus, c'est à dire le résidu ajouté aux solutions de lavage, sont dilués, filtrés à travers des filtres en nylon (0,2 µm) et analysés par chromatographie liquide (HPLC).

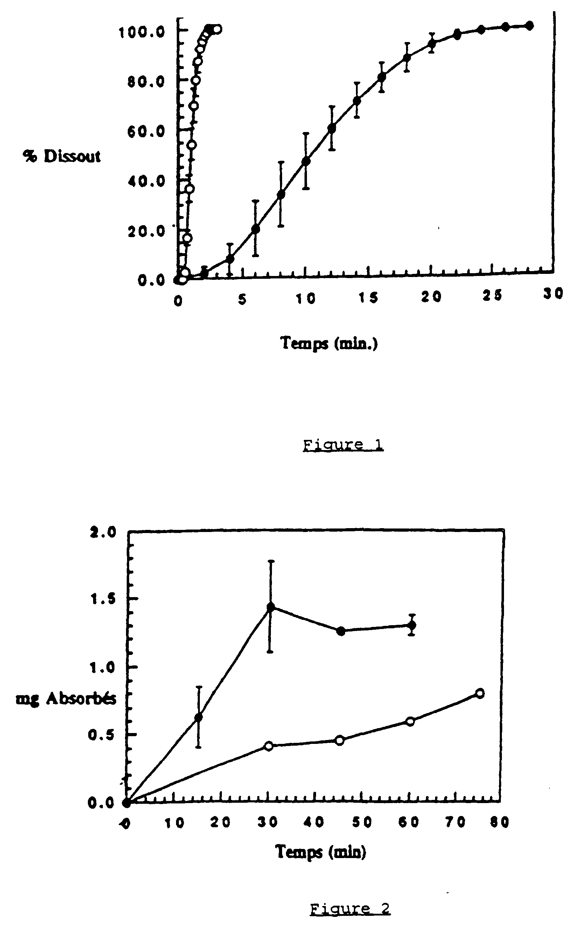

Les résultats sont donnés dans la figure 2.

### Exemple 4 : Essai d'imprégnation in vivo

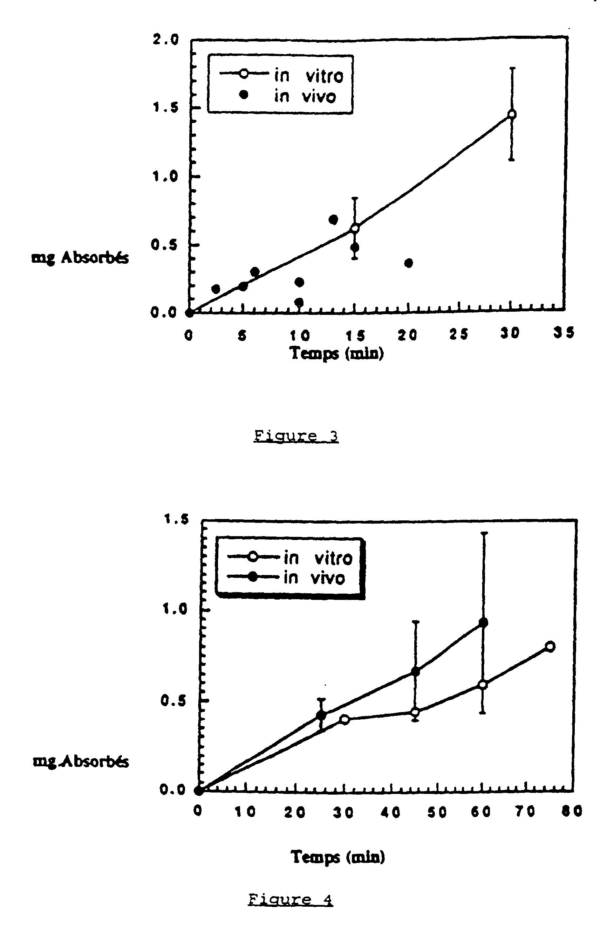

a) Les essais sur la composition à libération immédiate sont réalisés sur des volontaires sains, de sexe féminin d'un âge compris entre 26 et 34 ans.
   La bouche est rincée avec 100 ml d'eau distillée, la composition est par la suite placée sous la langue et gardée dans cette position sans déglutir pendant toute la durée de l'essai. Au terme du délai prévu, la salive est recueillie dans un bêcher et la bouche est à nouveau rincée avec de l'eau distillée (3x20 ml). La salive et les solutions de rinçage recueillies et portées à 200 ml avec de l'eau distillée dans un ballon étalonné, sont filtrées et analysée par chromatographie liquide (HPLC).
   Les résultats sont donnés dans la figure 3
b) Essais sur la composition à libération prolongée dans le temps
   La bouche est rincée avec environ 100 ml d'eau distillée. La composition est placée sur la muqueuse gingivale, dans le sillon entre la gencive et la partie intérieure de la lèvre. Au terme du délai fixé, pendant lequel la salive produite est aspirée et collectée, le résidu de la composition est recueilli et la bouche est rincée avec de l'eau distillée (2x20 ml). La salive et les solutions de rinçage mélangées et portées à 200 ml avec de l'eau distillée dans un ballon étalonné sont filtrées et analysés par HPLC.
   Les résultats sont donnés dans la figure 4.

Les figures ci-après illustrent les résultats.

La figure 1 illustre les profils de dissolution des deux compositions pharmaceutiques (libération immédiate (O); libération prolongée dans le temps (●) ; valeur moyenne ±sem; n = 3).

La mesure est effectuée à l'aide d'un appareil de dissolution à volume constant, constitué d'un bain thermostaté à 37 °C, relié à un spectrophotomètre U.V.-visible à travers une pompe péristaltique. Les vessels de dissolution contenant les compositions pharmaceutiques sont plongés dans 1000 ml d'eau à 37°C, sous agitation à 100 tours par minute, en utilisant la méthode de la palette rotative USP XIII. Le dosage du thiocolchicoside dissout est effectué par lecture spectrophotométrique.

La figure 2 compare les profils d'absorption du thiocolchicoside obtenus *in vitro* à travers la muqueuse buccale porcine en partant de compositions pharmaceutiques à libération immédiate (●) et à libération prolongée dans le temps (o).

La figure 3 présente la comparaison entre les quantités de thiocolchicoside absorbées *in vivo* (●) et *in vitro* (o) en partant d'une composition pharmaceutique à libération immédiate.(valeur moyenne ± sem ; n = 4)

La figure 4 présente la comparaison entre les quantités de thiocolchicoside absorbées *in vitro* (o) et *in vivo* (●) en partant d'une composition pharmaceutique à libération prolongée dans le temps.

Les résultats donnés dans les figures susmentionnées montrent que :
- Les profils de dissolution des deux compositions pharmaceutiques sont différents ; en effet, la composition à libération prolongée libère toute la quantité de thiocolchicoside dans un intervalle de temps de 30 minutes alors que la composition à libération immédiate la libère en 3 minutes ;
- Les compositions pharmaceutiques présentent de bonnes caractéristiques d'imprégnation à travers la muqueuse porcine (figure 2) et permettent un contrôle remarquable de la cinétique d'absorption du thiocolchicoside qui se différencie nettement pour ce qui est de la vitesse de dissolution et de la vitesse d'absorption.

De plus au cours des essais *in vivo,* les volontaires ont pu confirmer que la composition pharmaceutique à libération immédiate au contact de la muqueuse sublinguale provoque une discrète salivation et se dissout en donnant l'impression d'une disparition immédiate ; la composition pharmaceutique à libération prolongée dans le temps, au contact de la muqueuse buccale, s'hydrate lentement en formant une masse qui adhère à la muqueuse, sans provoquer localement de la gène ou de l'irritation et sans laisser de goût désagréable.

## Revendications

1. Composition pharmaceutique solide comprenant du thiocolchicoside adaptée pour l'administration à travers la muqueuse buccale de thiocolchicoside.

2. Composition pharmaceutique solide selon la revendication 1, **caractérisée en ce que** cette composition pharmaceutique comprend du thiocolchicoside de 4 à 20 mg par unité, de préférence de 4 à 8 mg par unité.

3. Composition pharmaceutique solide selon la revendication 2, **caractérisée en ce que** cette composition pharmaceutique comprend de la gélatine dans des proportions de 10 à 50% en masse.

4. Composition pharmaceutique à libération immédiate du thiocolchicoside selon l'une quelconque des revendications 1 à 3, **caractérisée en ce qu'**elle comprend un ou plusieurs excipients solubles dans des proportions de 30 à 70 % en masse.

5. Composition pharmaceutique à libération immédiate du thiocolchicoside selon la revendication 4, **caractérisée en ce que** le ou les excipients solubles sont choisis parmi la glycine ou des sucres tels que le mannitol, le sorbitol, le xylitol ou le glucose.

6. Composition pharmaceutique pour la libération prolongée du thiocolchicoside selon l'une des revendications 1 à 3, **caractérisée en ce qu'**elle comprend un ou plusieurs excipients gélifiables dans des proportions de 25 à 55 % en masse.

7. Composition pharmaceutique pour la libération prolongée du thiocolchicoside selon la revendication 6, **caractérisée en ce que** le ou les excipients gélifiables sont choisis parmi le collagène, la polyvinylpyrolidone, le polyéthylèneglycole, l'amidon soluble, la gomme de guar, la gomme de xanthane, l'alginate de sodium, les alcools polyvinyliques, la β-cyclodextrine et les dérivés de la cellulose comme la carboxyméthylcellulose sodique, l'hydroxypropyléthylcellulose ou l'hydroxypropylméthylcellulose.

8. Procédé de préparation des compositions pharmaceutiques solides selon l'une quelconques des revendications 1 à 7, **caractérisé en ce que** l'on lyophilise directement une solution contenant les composants de la composition pharmaceutique dans des bacs ayant des cavités préformées.

9. Procédé de préparation des compositions pharmaceutiques solides selon l'une quelconques des revendications 1 à 7, **caractérisé en ce que** l'on lyophilise en vrac une solution contenant les composants de la composition pharmaceutique pour donner un lyophilisé, on broie et tamise le lyophilisé ainsi obtenu pour donner une poudre, et on effectue une compression de la poudre ainsi obtenue.

10. Utilisation du thiocolchicoside pour la fabrication d'un médicament destiné à l'administration à travers la muqueuse buccale pour le traitement d'appoint des contractures musculaires douloureuses.

11. Composition pharmaceutique solide selon la revendication 1, **caractérisée en ce que** cette composition comprend : Thiocolchicoside : 4 mg
Gélatine : 14 mg
Glycine : 14 mg
Mannitol : 20 mg
Aspartame : 0,24 mg
Acide citrique : 1 mg et
Arome framboise : 0,5 mg.

12. Composition pharmaceutique solide selon la revendication 1, **caractérisée en ce que** cette composition comprend : Thiocolchicoside : 8 mg
Gélatine : 10 mg
Glycine : 14 mg
Mannitol : 20 mg
Aspartame : 0,5 mg
Acide citrique : 1 mg et
Arome framboise : 1 mg.

13. Composition pharmaceutique solide selon la revendication 1, **caractérisée en ce que** cette composition comprend : Thiocolchicoside 4 mg
Gélatine : 14 mg
Carboxyméthyle cellulose sodique : 12 mg
Aspartame : 0,3 mg
Acide citrique : 1 mg et
Arome framboise : 0,5 mg.

14. Composition pharmaceutique solide selon la revendication 1, **caractérisée en ce que** cette composition comprend : Thiocolchicoside : 8 mg
Gélatine : 10 mg
Carboxyméthyle cellulose : 12 mg
Aspartame : 0,4 mg
Acide citrique : 1 mg et
Arome framboise : 1 mg.

## Patentansprüche

1. Feste pharmazeutische Zubereitung enthaltend Thiocolchicosid zur Verabreichung von Thiocolchicosid über die Mundschleimhaut.

2. Feste pharmazeutische Zubereitung nach Anspruch 1, **dadurch gekennzeichnet, daß** diese pharmazeutische Zubereitung 4 bis 20 mg Thiocolchicosid pro Einheit, vorzugsweise 4 bis 8 mg pro Einheit, umfaßt.

3. Feste pharmazeutische Zubereitung nach Anspruch 2, **dadurch gekennzeichnet, daß** diese pharmazeutische Zubereitung Gelatine in Mengen von 10 bis 50 Massen-% umfaßt.

4. Pharmazeutische Zubereitung zur sofortigen Freisetzung von Thiocolchicosid nach einem der Ansprüche 1 bis 3, **dadurch gekennzeichnet, daß** sie ein oder mehrere lösliche Trägermaterialien in Mengen von 30 bis 70 Massen-% umfaßt.

5. Pharmazeutische Zubereitung zur sofortigen Freisetzung von Thiocolchicosid nach Anspruch 4, **dadurch gekennzeichnet, daß** das oder die löslichen Trägermaterialien aus Glycin oder Zucker, wie Mannitol, Sorbitol, Xylitol oder Glucose ausgewählt sind.

6. Pharmazeutische Zubereitung zur verzögerten Freisetzung von Thiocolchicosid nach einem der Ansprüche 1 bis 3, **dadurch gekennzeichnet, daß** sie ein oder mehrere gelierbare Trägermaterialien in Mengen von 20 bis 55 Massen-% umfaßt.

7. Pharmazeutische Zubereitung zur verzögerten Freisetzung von Thiocolchicosid nach Anspruch 6, **dadurch gekennzeichnet, daß** das oder die gelierbaren Trägermaterialien ausgewählt sind aus Collagen, Polyvinylpyrrolidon, Polyethylenglykol, löslicher Stärke, Guargummi, Xanthangummi, Natriumalginat, Polyvinylalkoholen, β-Cyclodextrin und Cellulosederivaten, wie Natrlumcarboxymethylcellulose, Hydroxypropylethylcellulose oder Hydroxypropylmethylcellulose.

8. Verfahren zur Herstellung der festen pharmazeutischen Zubereitungen nach einem der Ansprüche 1 bis 7, **dadurch gekennzeichnet, daß** man eine Lösung, welche die Bestandteile der pharmazeutischen Zubereitung enthält, in Behältern mit vorgebildeten Hohlräumen direkt gefriertrocknet.

9. Verfahren zur Herstellung der festen pharmazeutischen Zubereitungen nach einem der Ansprüche 1 bis 7, **dadurch gekennzeichnet, daß** man eine Lösung, welche die Bestandteile der pharmazeutischen Zubereitung enthält, in der Masse gefriertrocknet unter Bildung eines gefriergetrockneten Materials, worauf man das in dieser Weise erhaltene gefriergetrocknete Material zerkleinert und siebt zur Bildung eines Pulvers und das in dieser Weise erhaltene Pulver verpreßt.

10. Verwendung von Thiocolchicosid für die Herstellung eines Arzneimittels zur Verabreichung über die Mundschleimhaut bei der ergänzenden Behandlung von schmerzenden Muskelverspannungen.

11. Feste pharmazeutische Zubereitung nach Anspruch 1, **dadurch gekennzeichnet, daß** sie:
Thiocolchicosid: 4 mg
Gelatine: 14 mg
Glycin: 14 mg
Mannitol: 20 mg
Aspartam: 0,24 mg
Citronensäure: 1 mg und
Himbeeraroma: 0,5 mg
enthält.

12. Feste pharmazeutische Zubereitung nach Anspruch 1, **dadurch gekennzeichnet, daß** sie:
Thiocolchicosid: 8 mg
Gelatine: 10 mg
Glycin: 14 mg
Mannitol: 20 mg
Aspartam: 0,5 mg
Citronensäure: 1 mg und
Himbeeraroma: 1 mg
enthält.

13. Feste pharmazeutische Zubereitung nach Anspruch 1, **dadurch gekennzeichnet, daß** sie:
Thiocolchicosid: 4 mg
Gelatine: 14 mg
Natriumcarboxymethylcellulose: 12 mg
Aspartam: 0,3 mg
Citronensäure: 1 mg und
Himbeeraroma: 0,5 mg
enthält,

14. Feste pharmazeutische Zubereitung nach Anspruch 1, dadurch gekennzelchnet, daß sie:
Thiocolchicosid: 8 mg
Gelatine: 10 mg
Carboxymethylcellulose: 12 mg
Aspartam: 0,4 mg
Citronensäure: 1 mg und
Himbeeraroma: 1 mg
enthält.

## Claims

1. Solid pharmaceutical composition comprising thiocolchicoside suitable for the administration of thiocolchicoside via the buccal mucosa.

2. Solid pharmaceutical composition according to Claim 1, **characterized in that** this pharmaceutical composition comprises thiocolchicoside from 4 to 20 mg per unit, preferably from 4 to 8 mg per unit.

3. Solid pharmaceutical composition according to Claim 2, **characterized in that** this pharmaceutical composition comprises gelatine in proportions of 10 to 50% by mass.

4. Pharmaceutical composition for the immediate release of thiocolchicoside according to any one of Claims 1 to 3, **characterized in that** it comprises one or more soluble excipients in proportions of 30 to 70% by mass.

5. Pharmaceutical composition for the immediate release of thiocolchicoside according to Claim 4, **characterized in that** the soluble excipient(s) is/are chosen from glycine or sugars such as mannitol, sorbitol, xylitol or glucose.

6. Pharmaceutical composition for the prolonged release of thiocolchicoside according to one of Claims 1 to 3, **characterized in that** it comprises one or more gellable excipients in proportions of 25 to 55% by mass.

7. Pharmaceutical composition for the prolonged release of thiocolchicoside according to Claim 6, **characterized in that** the gellable excipient(s) is/are chosen from collagen, polyvinylpyrrolidone, polyethylene glycol, soluble starch, guar gum, xanthan gum, sodium alginate, polyvinyl alcohols, β-cyclodextrin and cellulose derivatives such as sodium carboxymethyl cellulose, hydroxypropyl ethyl cellulose or hydroxypropyl methyl cellulose.

8. Method for preparing the solid pharmaceutical compositions according to any one of Claims 1 to 7, **characterized in that** a solution containing the components of the pharmaceutical composition is freeze-dried directly in containers having preformed cavities.

9. Method for preparing the solid pharmaceutical compositions according to any one of Claims 1 to 7, **characterized in that** a solution containing the components of the pharmaceutical composition is freeze-dried in bulk to give a freeze-dried product, the freeze-dried product thus obtained is ground and sieved to give a powder, and the powder thus obtained is compressed.

10. Use of thiocolchicoside for the manufacture of a medicament for administration via the buccal mucosa for the adjuvant treatment of painful muscular contractures.

11. Solid pharmaceutical composition according to Claim 1, **characterized in that** this composition comprises:
Thiocolchicoside: 4 mg
Gelatine: 14 mg
Glycine: 14 mg
Mannitol: 20 mg
Aspartame: 0.24 mg
Citric acid: 1 mg and
Raspberry flavour: 0.5 mg.

12. Solid pharmaceutical composition according to Claim 1, **characterized in that** this composition comprises:
Thiocolchicoside: 8 mg
Gelatine: 10 mg
Glycine: 14 mg
Mannitol: 20 mg
Aspartame: 0.5 mg
Citric acid: 1 mg and
Raspberry flavour: 1 mg.

13. Solid pharmaceutical composition according to Claim 1, **characterized in that** this composition comprises:
Thiocolchicoside: 4 mg
Gelatine: 14 mg
Sodium carboxymethyl cellulose: 12 mg
Aspartame: 0.3 mg
Citric acid: 1 mg and
Raspberry flavour: 0.5 mg.

14. Solid pharmaceutical composition according to Claim 1, **characterized in that** this composition comprises:
Thiocolchicoside: 8 mg
Gelatine: 10 mg
Carboxymethyl cellulose: 12 mg
Aspartame: 0.4 mg
Citric acid: 1 mg and
Raspberry flavour: 1 mg.
